# EUROPEAN PATENT APPLICATION

(11) **EP 3 838 157 A1**
(43) Date of publication of application: **23.06.2021**
(21) Application number: 19218431.5
(22) Date of filing: 20.12.2019
(51) Int. Cl.: A61B 6/03, G06T 7/73, A61B 6/10, A61B 5/113, G16H 40/63, A61B 6/00

(54) **METHOD AND CALCULATING DEVICE FOR PROVIDING OBJECT DYNAMICS INFORMATION RELATING TO DYNAMICS OF AN OBJECT THAT IS ARRANGED ON A PATIENT HANDLING SYSTEM OF A MEDICAL IMAGING DEVICE**

(71) Applicant: Siemens Healthcare GmbH, 91052 Erlangen (DE)
(72) Inventor: Eschenbach, Tobias, 90762 Fürth (DE); Rossleben, Carsten, 91052 Erlangen (DE); Sühling, Michael, 91052 Erlangen (DE); Wimmer, Andreas, 91301 Forchheim (DE)

(57) **Abstract**

In one aspect the invention relates to a computer-implemented method for providing object dynamics information relating to dynamics of an object that is arranged on a patient handling system of a medical imaging device, the method comprising
- receiving sensor data relating to an outer contour of the object that is arranged on the patient handling system of the medical imaging device,
- calculating the object dynamics information based on the sensor data,
- providing the object dynamics information.

## Description

In one aspect the invention relates to a method for providing object dynamics information relating to dynamics of an object that is arranged on a patient handling system of a medical imaging device. In further aspects, the invention relates to a calculating device and to a medical imaging system.

Prior to performing a medical imaging examination (also referred to as "scan") with modalities such as computed tomography (CT) or magnetic resonance imaging (MRI), the patient is positioned on a table. The pose of the patient will depend on the examination to be performed. For example, patients undergoing a thorax examination are often placed in supine position on the table, either feet-first or head-first, with their arms placed behind their head in order to avoid occluding the examination region. In general, assuming and maintaining a certain pose is important in order to ensure the best image quality during the scan.

Therefore the pose of the patient will often be fixed with positioning aids such as head rests or knee rests in order to reduce the risk of accidental patient movement during the scan, which could lead to artifacts in the acquired images. Artifacts may also be caused by breathing motion if for instance the thorax is examined. Therefore the patient is often instructed to hold the breath or to follow specific breathing patterns.

As the patient table is moved to the start position for the medical scan, but also during or after the scan, a collision between the patient or equipment on the table and the scanner may happen. This problem typically occurs for heavier patients, for patients with lots of equipment on the table, or for patients where large positioning aids are used. Equipment is for instance often lying on the table in case of emergency patients, for example oxygen bottles or vital monitors. Large positioning aids are common in radiation therapy, e.g. breast boards. The patient may also be connected to an injector administering contrast agent that stands near the scanner.

A collision of the patient with the scanner leads to discomfort and may even cause injury to the patient. If equipment lying on the patient table collides with the scanner then the equipment or scanner may be damaged or may no longer work properly. For instance, the cables connecting a vital monitor to the patient might be disconnected because of the collision, or tubes for the administration of contrast agent may be pulled out.

In order to avoid collisions, the radiographer visually has to check if the patient fits into the gantry bore. This may require the radiographer to frequently stop the table as it is moved to the start position for the scan and to check for sufficient clearance between patient or equipment and the gantry bore. Even then it is unclear if a collision will occur at a later time, as the table is further moved into the gantry automatically during the scan. In order to be on the safe side, the user has to do a test run and manually move the table into the gantry for the whole planned scan range. This is time consuming and error prone. If a collision is to be expected, then the radiographer may choose to enter the gantry at a lower table height. This may lead to off centering, as mentioned above. If this does not avoid the collision, then the radiographer may have to reposition the patient or equipment in order to ensure the loaded table fits into the gantry. This is tedious for the patient and time consuming.

Maintaining a certain position during the medical scan is important in order to achieve the best image quality. Prior to the scan, patient movement may lead to the desired anatomical range along the horizontal axis of the table not being completely covered by the scan. Changing the vertical position, e.g. raising or lowering the head, may lead to a vertical misalignment, which in CT has adverse effects on image quality and dose (Reference [1]). During the medical scan, patient movement will usually lead to image artifacts.

In order to detect patient movement, the radiographer always needs to have an eye on the patient. However, during the medical scan, the radiographer usually has to leave the scan room, especially when ionizing radiation as in CT is released. During this time it is usually not possible to keep an eye on the patient. Regardless of the modality, during the scan, the radiographer will often not have the possibility to watch the patient inside the gantry.

Breathing movement, especially of the thorax, but also nearby body regions, may lead to artifacts in the acquired images. Therefore patients are usually instructed upfront to hold their breath or to follow specific breathing patterns. However, not all patients are able to follow these instructions.

The underlying technical problem of the invention is to facilitate an improved object dynamics information in the context of a medical imaging examination. This problem is solved by the subject matter of the independent claims. The dependent claims are related to further aspects of the invention.

In one aspect the invention relates to a computer-implemented method for providing object dynamics information relating to dynamics of an object that is arranged on a patient handling system of a medical imaging device, the method comprising
- receiving sensor data relating to an outer contour of the object that is arranged on the patient handling system of the medical imaging device,
- calculating the object dynamics information based on the sensor data,
- providing the object dynamics information.

The object can be, for example, a patient and/or medical equipment, in particular medical equipment that is connected to the patient. The object can be, for example, arranged on a patient transfer board of the patient handling system.

In another aspect calibration data regarding a calibration of a sensor system with respect to a coordinate system of the medical imaging device is received, the sensor system being configured for acquiring the sensor data, wherein the object dynamics information is calculated further based on the calibration data.

The sensor system can be calibrated to the coordinate system of the medical imaging device, for example, using a phantom, in particular a checkerboard phantom.

In another aspect the sensor data comprises at least one two-dimensional camera image and/or at least one three-dimensional camera image.

The sensor system can comprise, for example, at least one camera and/or at least one ultrasound-based range sensor. For example, one or more cameras can be mounted on the ceiling and/or on a gantry of the medical imaging device, in particular inside a tunnel-shaped opening of the gantry (also referred to as "gantry bore"), in which the patient can be positioned for the medical imaging examination. The cameras may be two-dimensional (2D) cameras or three-dimensional (3D) cameras or a combination of both. Here "3D camera" refers to a camera measuring the distances to objects based for instance on structured light or the time of flight principle.

In another aspect, the sensor system can comprise ultrasound range sensors and/or tactile sensors. Such sensors can be mounted, for example, around the tunnel-shaped opening, and/or can be employed to determine if there is sufficient clearance between the scanner and the patient or equipment that is arranged on the patient handling system of the medical imaging device.

In another aspect geometric shape data regarding a geometric shape of a gantry of the medical imaging device is received, wherein the object dynamics information is calculated further based on the geometric shape data. The geometric shape can be indicative, for example, of an outer contour of the medical imaging device and/or of an inner contour of a tunnel-shaped opening of the gantry of the medical imaging device.

If points or landmarks of the object lie outside the geometric shape describing the tunnel-shaped opening of the gantry bore, then they are likely to cause a collision.

The geometric shape can for example be a cylinder defined by the position and the diameter of the gantry bore. In case of a tilting gantry the geometric shape can be in form of an ellipsoid.

The extents of the patient, e.g. head, hands, elbows, hips, knees, feet, or the extents of equipment lying on the scanner table can be detected in combined 2D/3D camera images. Alternatively, a 3D point cloud can be acquired, and geometric constraints can be used to partition the scene into the table and surrounding objects. Instead of landmarks, one can also fit a virtual patient Avatar (Reference [2]) to the camera data and determine if the Avatar is fully inside the geometric shape or if an intersection occurs. In order to rule out false alerts, e.g. a radiographer standing next to the table reaching for the patient, algorithms to segment the scene into different entities and/or a more sophisticated scene understanding can be helpful.

In another aspect the object dynamics information comprises a collision information which relates to a collision of the object and a gantry of the medical imaging device, in particular when the gantry and the object move relative to each other.

The radiographer can be alerted of collisions, for example, by image, text, and/or audio notifications. The points of the object causing a collision can be visualized in the color image.

For example, an image of the scene with the patient and likely collision points can be presented to the radiographer. Thus the radiographer knows the critical areas before moving the table towards the gantry. Based on this information, the patient or equipment can be repositioned or a suitable table height not causing a collision can be chosen. Thereby collisions can be avoided, which may otherwise cause discomfort, stress or even injury to the patient. Thus, time is saved, and operator errors are avoided or at least reduced. Furthermore, damage to equipment arranged on the patient handling system or damage to the medical imaging device itself can be prevented.

In another aspect the object dynamics information comprises a motion information which relates to a motion of the object.

Motion detection can be performed, for example, by analyzing sensor data comprising 2D color streams or 3D depth streams or a combination of both. The changed regions of the object can be highlighted on a color image of the object, e.g. by marking the region where landmarks and/or limbs, in particular of the Avatar, have changed and/or where the optical flow is large and/or where the displacement field of an image registration takes large values.

If patient motion is detected, then the radiographer can be alerted to check if the patient position is still valid for the medical imaging examination. Detecting motion after the patient has been position by the radiographer but before the medical imaging examination has taken place helps to avoid scans where the patient is in an invalid position. This ensures the desired coverage and image quality.

In another aspect the calculating of the object dynamics information comprises calculating at least one motion-related dataset selected from the group consisting of an optical flow, a difference image, a displacement vector field, a landmark displacement dataset and a virtual patient model fitted to the sensor data, - wherein the motion information is calculated based on the at least one motion-related dataset.

The optical flow can be calculated, for example, based on a method described in Reference [3]. The difference image can be calculated, in particular, based on two consecutive frames. The displacement vector field can be calculated, in particular, based on image registration between consecutive video frames. Large displacement vectors are indicative of significant motion of the object, in particular of the patient. To separate patient movement from table movement, tracking body landmarks placed e.g. on joints and other salient body regions may be more sensitive (Reference [2]).

The landmark displacement dataset can be calculated, for example, by applying a machine learning-based landmark detection algorithm onto the sensor data. An example of a machine learning-based landmark detection algorithm is described in Reference [2]. The landmark displacement dataset can comprise, for each landmark of a plurality of landmarks in a 2D camera image and/or a 3D camera image, a respective displacement magnitude.

In another aspect, a virtual patient model in form of an Avatar can be fitted to the sensor data, in particular 3D depth data, in order to reliably track patient movement through Avatar movement, for example by analyzing the elevation and/or expansion of the chest area of the virtual patient model.

In another aspect the motion information comprises a motion compensation information, wherein a motion of the object is compensated based on the motion compensation information.

The motion compensation information can be, for example, in form of an adjustment information selected from the group consisting of a table position adjustment information, an examination protocol adjustment information and an image reconstruction parameter adjustment information.

If a system for automatic 2D/3D camera-based patient table positioning as described, for example, in Reference [4], is used and motion is detected, then the table position, especially the table height, can be automatically adjusted to counter the patient movement. If the patient movement cannot be compensated automatically by the system, then the radiographer can be alerted to check the patient pose and reposition, if necessary.

If motion is detected during the medical imaging examination, then the imaging data acquisition system and/or the image reconstruction system can account for the motion. If compensating the motion is not possible then the running scan can be aborted by the radiographer or by the system in order to prevent unnecessary dose exposure to the patient due to a not usable acquisition. If motion is detected but the impact on the image quality cannot be assessed during the scan then at least the radiographer can be alerted to immediately check the acquired images for artifacts and, if necessary, perform a new scan without losing time.

In another aspect the object is a patient, wherein the object dynamics information comprises a breathing information which relates to a breathing of the patient.

Breathing tracking can be performed in a manner similar to the motion detection described above, in particular by analyzing plain 2D color streams or 3D depth streams or a combination of both.
Breathing tracking can be used, for example, to train the patient and thus lead to a better preparation for the medical scan. The breathing parameters can moreover be used to train the right breathing patterns before an examination with an automatic verification.

In case of strong breathing motion, the radiographer can be alerted to perform a new scan immediately, if necessary. This saves time and costs, as setting up a separate appointment for a new examination is avoided. In another aspect, if strong breathing is detected then the radiographer may be alerted to check the acquired images for motion artifacts. In that case the scan may have to be repeated. Knowing this immediately during or after the initial scan attempt is beneficial, as the patient is still in the scan room and does not have to be called in for another scan at a later time.

The traditional approach to breathing tracking is equipping the patient with a breathing belt. Such equipment is costly, and it takes time to put the belt on and off. It also needs to be ensured that the belt material does not cause artifacts during the medical scan and is suitable for use e.g. in the magnetic field of an MR scanner. The proposed approach in contrast does not require the patient to wear special equipment.

In another aspect the calculating of the object dynamics information comprises calculating at least one breathing-related parameter selected from the group consisting of a breathing amplitude, a breathing frequency, a breathing phase labeling and a breathing volume, wherein the breathing information is calculated based on the at least one breathing-related parameter.

The breathing-related parameter, in particular the breathing amplitude, may be calculated, for example, based on the at least one motion-related dataset and/or based on tracking of distance values of points in a chest region, in particular when the sensor data comprises 3D depth data.

By analyzing the breathing amplitude over time the breathing frequency and/or the breathing phase labeling can be determined automatically. The breathing phase labeling can indicate, for example, an inspiration phase, an expiration and a breath hold. In addition, in particular when the sensor data comprises 3D depth data, an estimate of the breathing volume may be obtained by analyzing the expansion of the chest region, either directly in the depth image or based on the virtual patient model fitted to the sensor data.

Knowing the breathing frequency of a patient could allow the system to detect critical conditions indicated by abnormal breathing frequencies. Monitoring the breathing and especially the breathing frequency for abnormalities can be used to alert the radiographer of critical patient conditions. This is especially valuable if the radiographer has left the scan room to start an X-ray acquisition or when the radiographer cannot see the patient inside the gantry bore. Longer periods where the radiographer cannot see the patient occur for example during time consuming procedures like multiphase scans which may take several minutes in CT or hours for MI and MRT scans.

In another aspect the breathing information comprises a breathing compensation information, wherein a breathing of the patient is compensated based on the breathing compensation information.

The breathing compensation information can be, for example, in form of an examination protocol adjustment information and/or an image reconstruction parameter adjustment information.

The breathing parameters can be used to assess the breathing capabilities of the patient in order to select a suitable scan protocol (scan parameterization). Knowing the breathing capabilities of a patient may allow adjusting the scan protocol to better suit the individual patient. For example, the scan protocol can be automatically adjusted to the breathing capabilities of the patient. This leads to better image quality without artifacts and a better patient experience, because breathing commands that the patient is unable to follow can be avoided.

Furthermore, the breathing motion can be accounted for during the acquisition, e.g. by performing a gated scan. Thus, breathing artifacts during the scan can be avoided, resulting in better image quality.

In one further aspect the invention relates to a calculating device for providing object dynamics information relating to dynamics of an object that is arranged on a patient handling system of a medical imaging device, the calculating device comprising
- a sensor data receiver for receiving sensor data relating to an outer contour of the object that is arranged on the patient handling system of the medical imaging device,
- an object dynamics information calculator for calculating the object dynamics information based on the sensor data, and
- an object dynamics information provider for providing the object dynamics information.

In another aspect the calculating device is configured to implement the method according to one or more of the disclosed aspects.

In one further aspect the invention relates to a medical imaging system comprising
- a medical imaging device comprising a gantry and a patient handling system for positioning a patient with respect to the gantry,
- a sensor system for acquiring sensor data relating to an outer contour of an object that is arranged on the patient handling system,
- a calculating device according to one or more of the disclosed aspects for providing object dynamics information relating to dynamics of the object that is arranged on the patient handling system of the medical imaging device.

In one further aspect the invention relates to a computer program product comprising program elements which induce a calculating device to carry out the steps of the method according to one or more of the disclosed aspects, when the program elements are loaded into a memory of the calculating device.

In another aspect a computer-readable medium is hereby disclosed on which program elements are stored that can be read and executed by a calculating device, in order to perform the steps of the method according to one or more of the disclosed aspects, when the program elements are executed by the calculating device.

The computer program product can be, for example, a computer program or comprise another element apart from the computer program. This other element can be hardware, for example a memory device, on which the computer program is stored, a hardware key for using the computer program and the like, and/or software, for example, a documentation or a software key for using the computer program. A computer-readable medium can be embodied as non-permanent main memory (e.g. random-access memory) or as permanent mass storage (e.g. hard disk, USB stick, SD card, solid state disk).

The calculating device can comprise, for example, at least one of a cloud-computing system, a distributed computing system, a computer network, a computer, a tablet computer, a smartphone or the like. The calculating device can comprise hardware and/or software. The hardware can be, for example, a processor, a computer-readable medium or a combination thereof. The hardware can be configurable by the software and/or be operable by the software. Calculations for performing an action of a method may be carried out in a processor.

Data, in particular the sensor data, the calibration data and/or the geometric shape data, can be received, for example, by receiving a signal that carries the data and/or by reading the data from a computer-readable medium and/or by receiving an input through a user interface. Data, in particular the object dynamics information, can be provided, for example, by transmitting a signal that carries the data and/or by writing the data into a computer-readable medium and/or by displaying the data on a display.

Any of the algorithms mentioned herein can be based on one or more of the following architectures: convolutional neural network, deep belief network, random forest, deep residual learning, deep reinforcement learning, recurrent neural network, Siamese network, generative adversarial network or auto-encoder. In particular, a trained machine learning algorithm can be embodied as a deep learning algorithm, in particular as a deep convolutional neural network.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature.

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention

Reference is made to the fact that the described methods and the described system are merely preferred example embodiments of the invention and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a patient being arranged for a medical imaging examination on the patient handling system.
Fig. 2 shows a two-dimensional camera color image and a depth image of a patient.
Fig. 3 shows examples for collision detection using a combined color/depth camera.
Fig. 4 shows a depth image with points in the chest/abdomen region that are tracked over time.
Fig. 5 shows a diagram illustrating a computer-implemented method for providing object dynamics information of an object.
Fig. 6 shows a calculating device for providing object dynamics information relating to dynamics of an object.
Fig. 7 shows a medical imaging system.

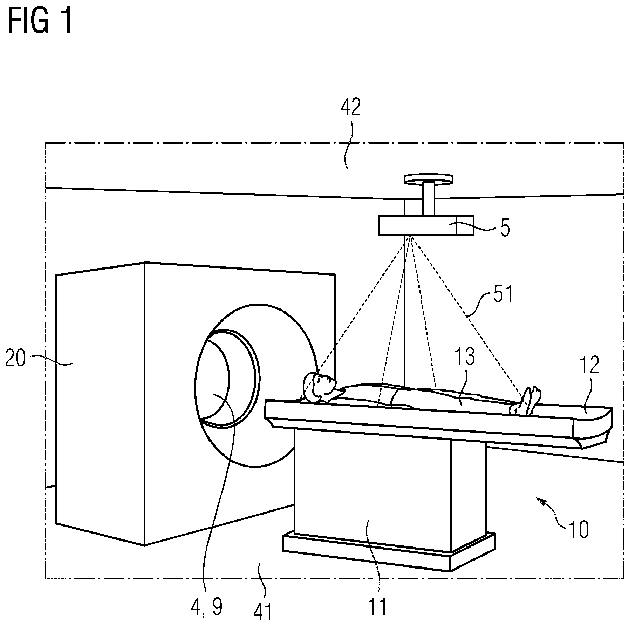

Fig. 1 shows a patient 13 being arranged for a medical imaging examination on the patient handling system 10. A sensor system in form of the 2D/3D camera system 5 mounted on the ceiling 42 acquires images of the patient 13 that can be used for collision detection, patient movement detection, and/or breathing tracking. The camera system 5 has the field of view 51.

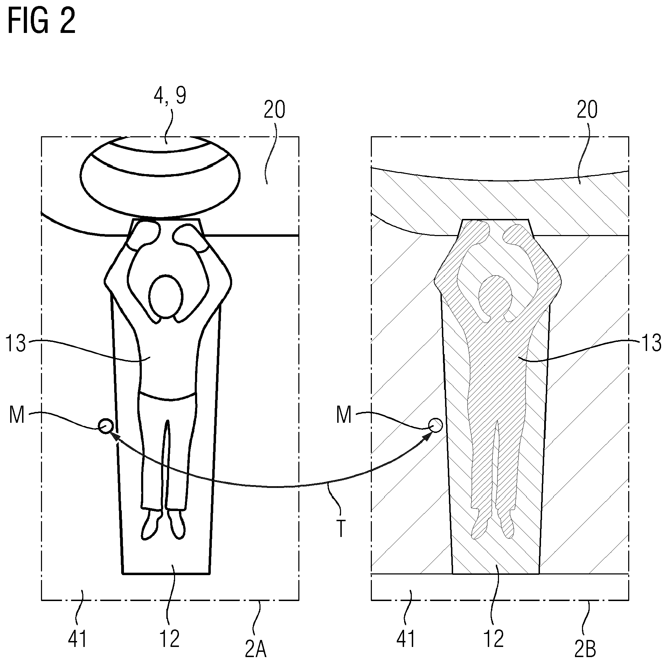

Fig. 2 shows a two-dimensional camera color image 2A (color not shown) and a depth image 2B of a patient 13 lying on the table 12 of the patient handling system 10.

The grey values in the depth image 2B indicate the distance of objects to the camera 5. A darker color means the object is closer, brighter means the object is further away, for example near the floor 41. Regions shown in white are undefined, e.g. because of different field-of-views of the cameras. The depth image 2B has been mapped onto the two-dimensional camera image 2A using a transform T that is based on the calibration of each camera of the camera system 5 to the coordinate system of the medical imaging device 1. Alternatively, the cameras could be calibrated pairwise to each other and one camera could be calibrated to the coordinate system of the medical imaging device 1.

Since all cameras are calibrated to the same coordinate system, a mapping T from one camera image to another can be established. The mapping T between the camera images allows highlighting points, for example point M, detected in one camera image, e.g. the depth image 2B, in another camera image that may be more natural to a human operator, e.g. the image 2A. This can be useful when a critical event such as patient motion is detected in a depth image. The event can then be visualized at the corresponding location in the color image, which is more natural to understand for a human operator.

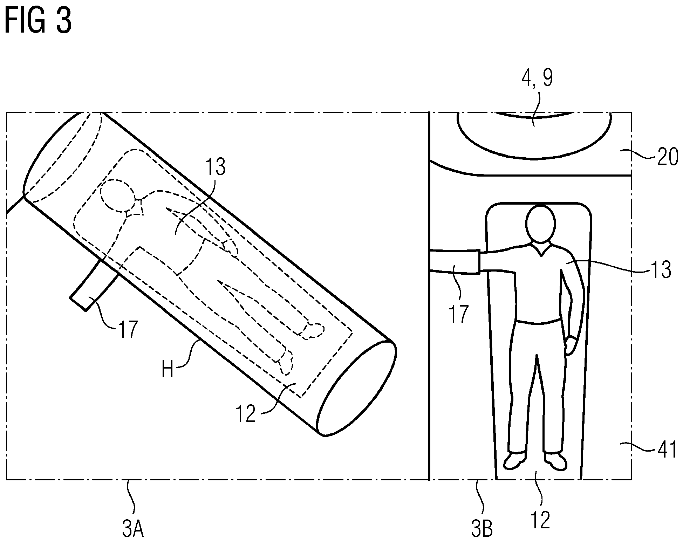

Fig. 3 shows examples for collision detection using a combined color/depth camera. In the 3D scene 3A a point cloud acquired with the depth camera is shown. In this example, the gantry bore is modeled with a cylinder H defined by the position and the diameter of the gantry bore 9. All points outside the cylinder H that are connected to structures inside the cylinder H, i.e. to the moving table 12, are highlighted with a marking 17, for example, based on a color-code.

The collision detection result can be presented to the user in by highlighting critical regions in the corresponding color image with a marking 17, for example, in form of an overlay by a colored area.

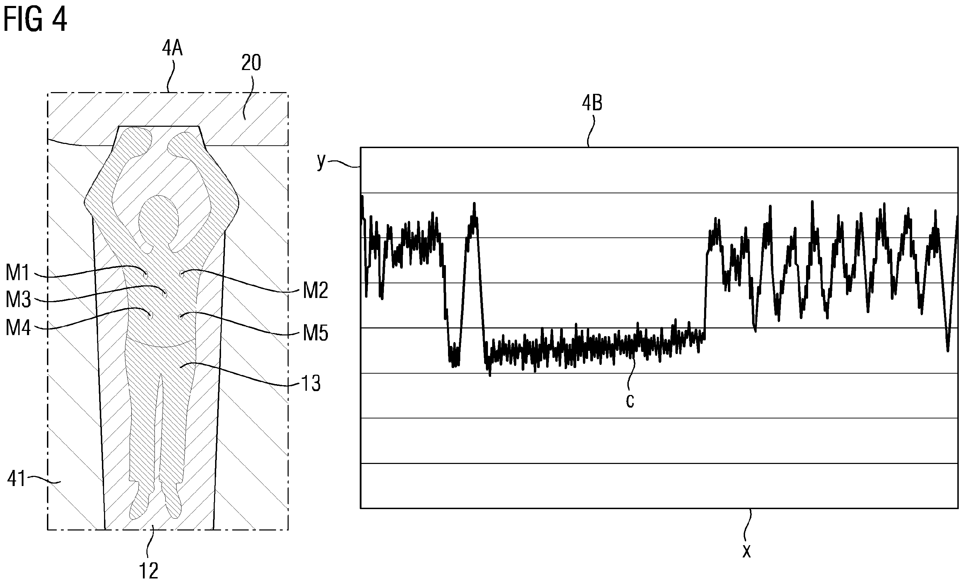

Fig. 4 shows a depth image 2B with points M1, M2, M3, M4 and M5 in the chest/abdomen region that are tracked over time. The graph 2B shows the resulting breathing curve over the acquired frames (time). The breathing curve is calculated based on corresponding distances between the points M1, M2, M3, M4 and M5.

The central flat segment denotes a phase of breath hold. The following periodic signal describes regular breathing. The breathing frequency can be calculated from the period length, considering the acquisition times of the frames, whereas the amplitude gives insight into the breathing depth or volume.

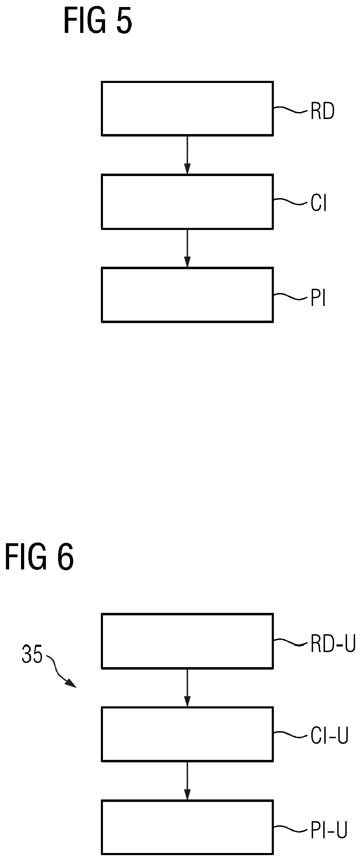

Fig. 5 shows a diagram illustrating a computer-implemented method for providing object dynamics information relating to dynamics of an object, in particular in form of the patient 13, that is arranged on a patient handling system 10 of a medical imaging device 1, the method comprising
- receiving RD sensor data relating to an outer contour of the object 13 that is arranged on the patient handling system 10 of the medical imaging device 1,
- calculating CI the object dynamics information based on the sensor data,
- providing PI the object dynamics information.

Fig. 6 shows a calculating device 35 for providing object dynamics information relating to dynamics of an object, in particular in form of the patient 13, that is arranged on a patient handling system 10 of a medical imaging device 1, the calculating device 35 comprising:
- a sensor data receiver RD-U for receiving RD sensor data relating to an outer contour of the object 13 that is arranged on the patient handling system 10 of the medical imaging device 1,
- an object dynamics information calculator CI-U for calculating CI the object dynamics information based on the sensor data, and
- an object dynamics information provider PI-U for providing PI the object dynamics information.

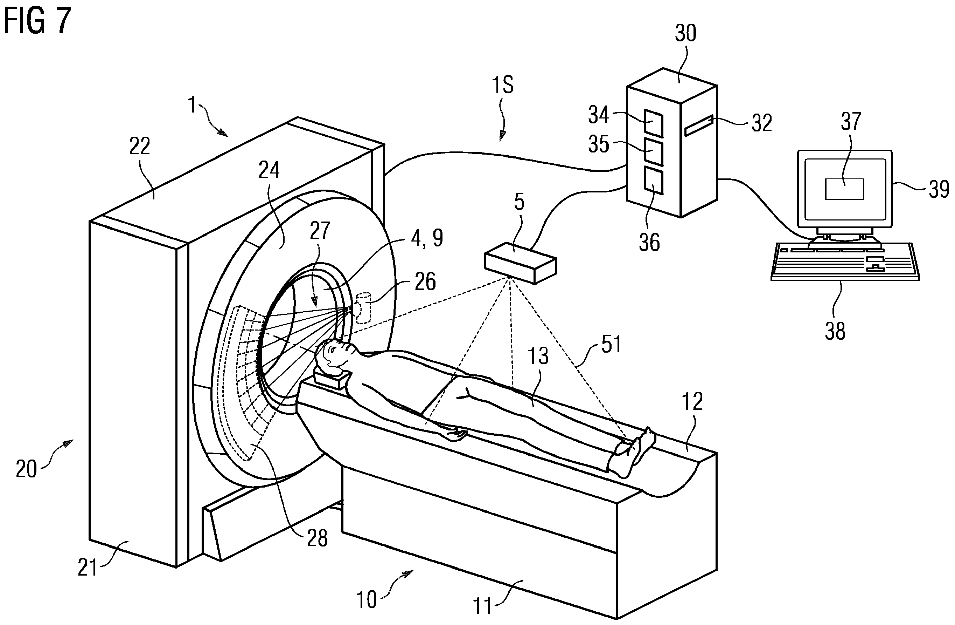

Fig. 7 shows a medical imaging system 1S, comprising:
- the medical imaging device 1 in form of a computed tomography device comprising a gantry 20 and a patient handling system 10 for positioning a patient 13 with respect to the gantry 20,
- the sensor system 5 for acquiring sensor data relating to an outer contour of an object, in particular the patient 13, that is arranged on the patient handling system 10,
- the calculating device 35 according for providing object dynamics information relating to dynamics of the object 13 that is arranged on the patient handling system 10 of the medical imaging device 1.

The gantry 20 comprises a support frame 21, a tilting frame 22, and a rotating frame 24, an x-ray source 26, an x-ray detector 28. The patient handling system 10 comprises a base 11 and a patient transfer board 12. The patient handling system 10 is configured for placing the patient 13, by a translation movement of the transfer board 12 with respect to the base 11, in the tunnel-shaped opening 9 of the gantry 20. Thereby the area of interest of the patient 13 can be placed in the acquisition portion 4 of the computed tomography device 1 for interaction with the x-rays 27 emitted from the x-ray source 26. The x-ray source 26 and the x-ray detector 28 are mounted on the rotating frame 24 for rotation around the acquisition portion 4 located within the tunnel-shaped opening 9.

The computed tomography device 1 further comprises a control unit 30, the control unit 30 comprising the computer-readable medium 32, the image reconstruction unit 34 for reconstructing a medical image based on the projection data, the calculating device 35 and the processor 36, an input unit 38 (e.g. a keyboard and/or a mouse) and a display 39 for displaying, for example medical images, examination parameters and/or alerts 37, in particular motion-related and/or breathing related alerts.

### References

[1] Prevalence and Severity of Off-Centering During Diagnostic CT: Observations From 57,621 CT scans of the Chest, Abdomen, and/or Pelvis, Akin-Akintayo OO, Alexander LF, Neill R, Krupinksi EA, Tang X, Mittal PK, Small WC, Moreno CC, Current Problems in Diagnostic Radiology, 2018
[2] DARWIN: Deformable Patient Avatar Representation with Deep Image Network, Singh, Vivek & Ma, Kai & Tamersoy, Birgi & Chang, Yao-Jen & Wimmer, Andreas & O'Donnell, Thomas & Chen, Terrence, MICCAI, 2017, 497-504.
[3] Determining optical flow, Berthold K. P. Horn, Brian G. Schunck, Artificial Intelligence, 17 (1-3), 1981, 185-203.
[4] Determining a value of a recording parameter by use of an anatomic landmark,
   Thomas Flohr, Michael Scheuering, Bernhard Schmidt, Martin Sedlmair, Grzegorz Soza, US 2015/0104092 A1.

## Claims

1. Computer-implemented method for providing object dynamics information relating to dynamics of an object (13) that is arranged on a patient handling system (10) of a medical imaging device (1), the method comprising
- receiving (RD) sensor data relating to an outer contour of the object (13) that is arranged on the patient handling system (10) of the medical imaging device (1),
- calculating (CI) the object dynamics information based on the sensor data,
- providing (PI) the object dynamics information.

2. Method according to claim 1,
- wherein calibration data regarding a calibration of a sensor system (5) with respect to a coordinate system of the medical imaging device (1) is received, the sensor system (5) being configured for acquiring the sensor data,
- wherein the object dynamics information is calculated further based on the calibration data.

3. Method according to claim 1 or 2,
- wherein the sensor data comprises at least one two-dimensional camera image and/or at least one three-dimensional camera image.

4. Method according to one of the claims 1 to 3,
- wherein geometric shape data regarding a geometric shape of a gantry (20) of the medical imaging device (1) is received,
- wherein the object dynamics information is calculated further based on the geometric shape data.

5. Method according to one of the claims 1 to 4,
- wherein the object dynamics information comprises a collision information which relates to a collision of the object (13) and a gantry (20) of the medical imaging device (1).

6. Method according to one of the claims 1 to 5,
- wherein the object dynamics information comprises a motion information which relates to a motion of the object (13).

7. Method according to claim 6,
- wherein the calculating of the object dynamics information comprises calculating at least one motion-related dataset selected from the group consisting of an optical flow, a difference image, a displacement vector field, a landmark displacement dataset and a virtual patient model fitted to the sensor data,
- wherein the motion information is calculated based on the at least one motion-related dataset.

8. Method according to claim 6 or 7,
- wherein the motion information comprises a motion compensation information,
- wherein a motion of the object (13) is compensated based on the motion compensation information.

9. Method according to one of the claims 1 to 8,
- wherein the object (13) is a patient,
- wherein the object dynamics information comprises a breathing information which relates to a breathing of the patient.

10. Method according to claim 9,
- wherein the calculating of the object dynamics information comprises calculating at least one breathing-related parameter selected from the group consisting of a breathing amplitude, a breathing frequency, a breathing phase labeling and a breathing volume,
- wherein the breathing information is calculated based on the at least one breathing-related parameter.

11. Method according to claim 9 or 10,
- wherein the breathing information comprises a breathing compensation information,
- wherein a breathing of the patient is compensated based on the breathing compensation information.

12. Calculating device for providing object dynamics information relating to dynamics of an object (13) that is arranged on a patient handling system (10) of a medical imaging device (1), the calculating device comprising
- a sensor data receiver (RD-U) for receiving (RD) sensor data relating to an outer contour of the object (13) that is arranged on the patient handling system (10) of the medical imaging device (1),
- an object dynamics information calculator (CI-U) for calculating (CI) the object dynamics information based on the sensor data, and
- an object dynamics information provider (PI-U) for providing (PI) the object dynamics information.

13. Calculating device (35) according to claim 12, configured to implement the method of one of the claims 1 to 11.

14. A medical imaging system (IS) comprising
- a medical imaging device (1) comprising a gantry (20) and a patient handling system (10) for positioning a patient with respect to the gantry (20),
- a sensor system (5) configured for acquiring sensor data relating to an outer contour of an object (13) that is arranged on the patient handling system (10),
- a calculating device (35) according to claim 12 or 13 for providing object dynamics information relating to dynamics of the object (13) that is arranged on the patient handling system (10) of the medical imaging device (1).

15. Computer program product comprising program elements which induce a calculating device (35) to carry out the steps of the method according to one of the claims 1 to 11, when the program elements are loaded into a memory of the calculating device (35).
